# EUROPEAN PATENT APPLICATION

(11) **EP 4 116 284 A1**
(43) Date of publication of application: **11.01.2023**
(21) Application number: 20922821.2
(22) Date of filing: 22.12.2020
(51) Int. Cl.: C07C 39/15, C08G 65/332, C08G 65/333, C07C 37/00

(54) **POLYROTAXANE, METHOD FOR MANUFACTURING SAME, AND CROSSLINKED POLYROTAXANE**

(30) Priority: 03.03.2020 JP 2020036286
(71) Applicant: Toyoda Gosei Co., Ltd., Kiyosu-shi, Aichi 452-8564 (JP); Kyoto University, Kyoto-shi, Kyoto 606-8501 (JP); National University Corporation Kanazawa University, Ishikawa 920-1192 (JP)
(72) Inventor: TAMAI Hideki, Kiyosu-shi, Aichi 452-8564 (JP); NAKAI Takanori, Kiyosu-shi, Aichi 452-8564 (JP); IMAI Hideyuki, Kiyosu-shi, Aichi 452-8564 (JP); ISHIDA Makoto, Kiyosu-shi, Aichi 452-8564 (JP); OGOSHI Tomoki, Kyoto-shi, Kyoto 606-8501 (JP); MAEDA Koki, Kyoto-shi, Kyoto 606-8501 (JP); ONISHI Katsuto, Kyoto-shi, Kyoto 606-8501 (JP); YAMAGISHI Tada-Aki, Kanazawa-shi, Ishikawa 920-1192 (JP); KAKUTA Takahiro, Kanazawa-shi, Ishikawa 920-1192 (JP); YAGYU Masafumi, Kanazawa-shi, Ishikawa 920-1192 (JP)
(74) Representative: Kramer Barske Schmidtchen Patentanwälte PartG mbB
(86) International application number: PCT/JP2020/047967
(87) International publication number: WO 2021/176809

(57) **Abstract**

The present invention provides a polyrotaxane of high heat resistance. The polyrotaxane contains a linear molecule, a cyclic molecule enclosing the linear molecule such that the cyclic molecule is skewered with the linear molecule, and blocking groups disposed at both ends of the linear molecule. The cyclic molecule contains an aromatic ring having, on a side chain, a phenolic hydroxyl group. The polyrotaxane can be produced by a method including a step of dissolving, in a methanol-containing solvent, a linear molecule and a cyclic molecule containing an aromatic ring having, on a side chain, a phenolic hydroxyl group, to yield a pseudo polyrotaxane in which the cyclic molecule encloses the linear molecule such that the cyclic molecule is skewered with the linear molecule; and a step of dissolving the pseudo polyrotaxane and a blocking group material in a solvent, to dispose blocking groups at both ends of the linear molecule.

## Description

### TECHNICAL FIELD

The present invention relates to a polyrotaxane.

### BACKGROUND ART

A polyrotaxane is a molecular assembly having a structure containing a linear molecule, a cyclic molecule enclosing the linear molecule such that the cyclic molecule is skewered with the linear molecule (i.e., the linear molecule is inserted through the hole of the cyclic molecule), and blocking groups disposed at both ends of the linear molecule. Since the cyclic molecule is slidable with respect to the linear molecule, the polyrotaxane is called "slide-ring material (SRM) ." Various cyclic molecules and linear molecules are known. Generally, cyclodextrin is used as a cyclic molecule, and polyethylene glycol is used as a linear molecule (Patent Documents 1 and 2).

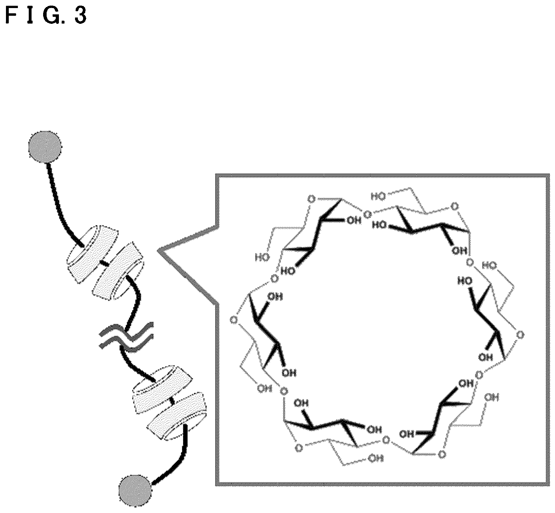

Cyclodextrin has a structure in which D-glucose molecules are connected in a ring form. FIG. 3 shows the structural formula of α-cyclodextrin having six D-glucose ring members. Cyclodextrin has many hydroxyl groups at ends of a hole, and oxygen atoms and hydrogen atoms of ether bonds in the hole.

### PRIOR ART DOCUMENTS

### Patent Documents

Patent Document 1: International Publication WO 2005/080469
Patent Document 2: International Publication WO 2018/038124
Patent Document 3: International Publication WO 2019/208723

### Non-Patent Documents

Non-Patent Document 1: Tomoki Ogoshi, et al., "Facile, Rapid, and High-Yield Synthesis of Pillar[5]arene from Commercially Available Reagents and Its X-ray Crystal Structure" JOC 2011, 76, pp. 328-331

### SUMMARY OF THE INVENTION

### Problems to be Solved by the Invention

However, according to studies by the present inventors, a polyrotaxane containing cyclodextrin as a cyclic molecule has a problem of low heat resistance.

Thus, an object of the present invention is to provide a polyrotaxane of high heat resistance.

Patent Document 3 discloses a rotaxane compound wherein the cyclic molecule is selected from the group consisting of, for example, cyclodextrin, pillararene, and calixarene. Since the rotaxane compound serves as a silane coupling agent, the compound has a functional group that binds to an organic material and a functional group that binds to an inorganic material. However, this document does not describe that pillararene or calixarene has, on its side chain, a phenolic hydroxyl group.

Non-Patent Document 1 discloses synthesis of phenolic pillararene. However, this document does not describe, for example, application of the pillararene to a polyrotaxane, and the purpose and effect thereof.

### Means for Solving the Problems

### [1] Polyrotaxane

A polyrotaxane containing a linear molecule, a cyclic molecule enclosing the linear molecule such that the cyclic molecule is skewered with the linear molecule, and blocking groups disposed at both ends of the linear molecule, characterized in that:
the cyclic molecule contains an aromatic ring having, on a side chain, a phenolic hydroxyl group.

### (Effects)

Since the cyclic molecule contains an aromatic ring having, on its side chain, a phenolic hydroxyl group, the polyrotaxane exhibits improved heat resistance.

### [2] Production Method for Polyrotaxane

A method for producing a polyrotaxane, the method including:
a step of dissolving, in a methanol-containing solvent, a linear molecule and a cyclic molecule containing an aromatic ring having, on a side chain, a phenolic hydroxyl group, to yield a pseudo polyrotaxane in which the cyclic molecule encloses the linear molecule such that the cyclic molecule is skewered with the linear molecule; and
a step of dissolving the pseudo polyrotaxane and a blocking group material in a solvent, to dispose blocking groups at both ends of the linear molecule.

The methanol-containing solvent is preferably a mixed solvent of methanol and water.

### (Effects)

When a linear molecule and a cyclic molecule containing an aromatic ring having, on its side chain, a phenolic hydroxyl group are dissolved in a methanol-containing solvent, a pseudo polyrotaxane in which the cyclic molecule encloses the linear molecule such that the cyclic molecule is skewered with the linear molecule is synthesized and precipitated.

In contrast, when the solvent is only water, the cyclic molecule does not dissolve in the solvent (see the following Table 1) .

When the solvent is only methanol, the cyclic molecule dissolves in the solvent, but the pseudo polyrotaxane does not precipitate therein. Conceivably, this phenomenon is attributed to the fact that the pseudo polyrotaxane cannot be synthesized when the cyclic molecule dissolves excessively in the solvent. A similar phenomenon occurs when the solvent is, for example, ethanol or acetone.

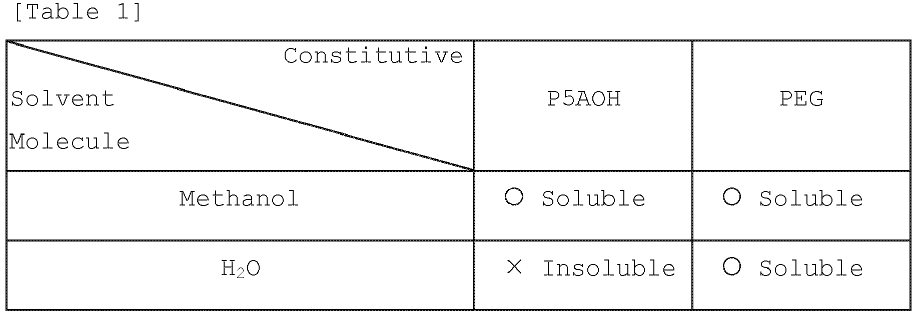

### [3] Crosslinked Polyrotaxane

A crosslinked polyrotaxane produced through crosslinking between cyclic molecules of a plurality of the polyrotaxanes according to [1] above by means of a crosslinking agent.

### [4] Elastomer

An elastomer containing the crosslinked polyrotaxane according to [3] above.

No particular limitation is imposed on the application of the elastomer. For example, an electrode may be attached to the elastomer, and the resultant product may be used as a polymer actuator or a polymer sensor.

### Effects of the Invention

The present invention can provide a polyrotaxane and a crosslinked polyrotaxane of high heat resistance.

### BRIEF DESCRIPTION OF THE DRAWINGS

FIG. 1 is a schematic view for describing a production process for a polyrotaxane of Example;
FIG. 2 is a schematic view of the polyrotaxane of Example; and
FIG. 3 is a schematic view of a polyrotaxane of Comparative Example.

### MODES FOR CARRYING OUT THE INVENTION

### 1. Polyrotaxane

### (a) Cyclic Molecule (cyclic molecule containing an aromatic ring having, on its side chain, a phenolic hydroxyl group)

Examples of the aromatic ring include a benzene ring, a naphthalene ring, and an anthracene ring.

Examples of the cyclic molecule include pillararene or calixarene having, on its side chain, a phenolic hydroxyl group.

In the cyclic molecule, a portion of the phenolic hydroxyl group of the side chain may be substituted with another group, such as -SH, -NH₂, -COOH, -SO₃H, or -PO₄H. Alternatively, a portion of the phenolic hydroxyl group may be substituted with a substituent having a graft chain (e.g., a graft chain prepared by ring-opening polymerization of a lactone monomer) so that the cyclic molecule can be dissolved in various organic solvents.

Pillararene is an oligomer having a structure in which arenes (aromatic rings) are connected in a cyclic and prismatic form. In general, pillararene is represented as pillar[n]arene (wherein [n] is the number of arene rings) . The number [n], which is not particularly limited, is preferably 5 to 6.

Calixarene is an oligomer having a structure in which phenol molecules are connected in a cyclic form via methylene groups. In general, calixarene is represented as calix[n]arene (wherein [n] is the number of phenol rings) . The number [n], which is not particularly limited, is preferably 3 to 10.

### (b) Linear Molecule

Examples of the linear molecule include, but are not particularly limited to, polyethylene glycol, polylactic acid, polyisoprene, polyisobutylene, polybutadiene, polypropylene glycol, polytetrahydrofuran, polydimethylsiloxane, polyethylene, polypropylene, polyvinyl alcohol, and polyvinyl methyl ether. The linear molecule is preferably polyethylene glycol, and may contain polyethylene glycol and another linear molecule.

### (c) Blocking Group

Examples of the blocking group include, but are not particularly limited to, dinitrophenyl group, cyclodextrin group, adamantane group, trityl group, fluorescein group, pyrene group, substituted benzene group (the substituent may be, for example, alkyl, alkyloxy, hydroxy, halogen, cyano, sulfonyl, carboxyl, amino, or phenyl; one or more substituents may be present), optionally substituted polynuclear aromatic group (the substituent may be, for example, the same as those described above; one or more substituents may be present), and steroid group. The blocking group is preferably selected from the group consisting of dinitrophenyl group, cyclodextrin group, adamantane group, trityl group, fluorescein group, and pyrene group, and is more preferably adamantane group or trityl group.

### 2. Crosslinking Agent

Examples of the crosslinking agent for the polyrotaxane include, but are not particularly limited to, isocyanate, polyether, polyester, polysiloxane, polycarbonate, poly (meth) acrylate or polyene, or copolymers thereof, or mixtures thereof.

No particular limitation is imposed on the functional group disposed at each end of the crosslinking agent. The functional group is preferably an isocyanate group capable of reacting with the phenolic hydroxyl group of the cyclic molecule, more preferably a blocked isocyanate group.

### 3. Elastomer

The elastomer may be composed only of a crosslinked polyrotaxane, or may be a mixture of a crosslinked polyrotaxane and an additional elastomer, etc.

Examples of the additional elastomer include, but are not particularly limited to, silicone elastomer, styrenic thermoplastic elastomer, natural rubber, nitrile rubber, acrylic rubber, urethane rubber, urea rubber, and fluororubber.

### Examples

The present invention will next be described with reference to Example and Comparative Example.

### [Example]

The polyrotaxane of Example was produced by a method including the following steps (1) to (4).

### (1) Activation of Both Ends of Polyethylene Glycol (abbreviated as "PEG")

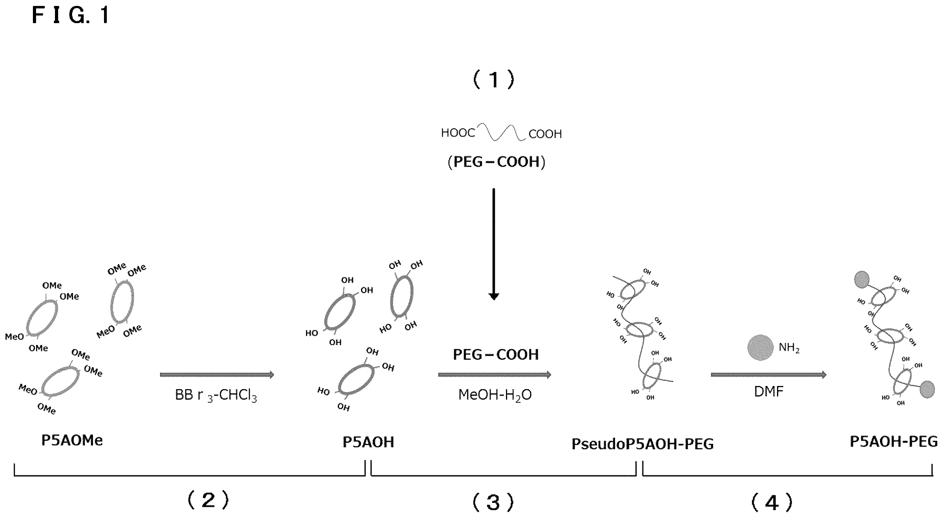

As shown in FIG. 1(1), according to the method described in the literature (Macromolecules, 2005, 38, 7524-7527), 2,2,6,6-tetramethylpiperidine 1-oxyl (TEMPO), NaBr, and NaClO were added to an aqueous solution of polyethylene glycol (PEG 20000), and reaction was allowed to proceed at pH of 10 to 11 for 15 minutes. The reaction mixture was treated with diluted hydrochloric acid, and the resultant mixture was subjected to extraction twice with dichloromethane. The resultant dichloromethane solution was recovered and concentrated under reduced pressure, followed by recrystallization with ethanol, to thereby prepare polyethylene glycol having carboxyl groups at both ends of the molecule (hereinafter abbreviated as "PEG-COOH") at a weight yield of 91%.

### (2) Synthesis of Pillar[5]arene

As shown in FIG. 1(2), according to the method described in the literature (J. Org. Chem. 2011, 76, 328-331), 13.6 g (54.3 mmol) of boron tribromide was added to a solution of 2.00 g (2.67 mmol) of dimethoxypillar[5]arene in 150 mL of anhydrous chloroform, and the mixture was stirred at 25°C for 72 hours. Water was added to the reaction mixture, and the resultant precipitate was recovered. The precipitate was washed with 0.5 M aqueous HCl solution and chloroform, to thereby quantitatively prepare 1.61 g (2.64 mmol) of pillar[5]arene containing an aromatic ring having, on its side chain, a phenolic hydroxyl group (hereinafter abbreviated as "P5AOH").

### (3) Synthesis of Pseudo Polyrotaxane

As shown in FIG. 1(3), 10 mL (0.0121 mol/L) of P5AOH solution prepared by using 10 mL of aqueous methanol solution (mixing ratio by weight of methanol : water = 1 : 1) as a solvent was mixed with 0.6 mL (1.894 mol/L) of PEG-COOH solution, and the mixture was allowed to stand still at room temperature for one day. The resultant precipitate was washed with 10 mL of water, and the resultant residue was dried under vacuum at 50°C for one day, to thereby produce a pseudo polyrotaxane in which P5AOH encloses PEG-COOH (hereinafter abbreviated as "Pseudo P5AOH-PEG").

### (4) Synthesis of Polyrotaxane of Example

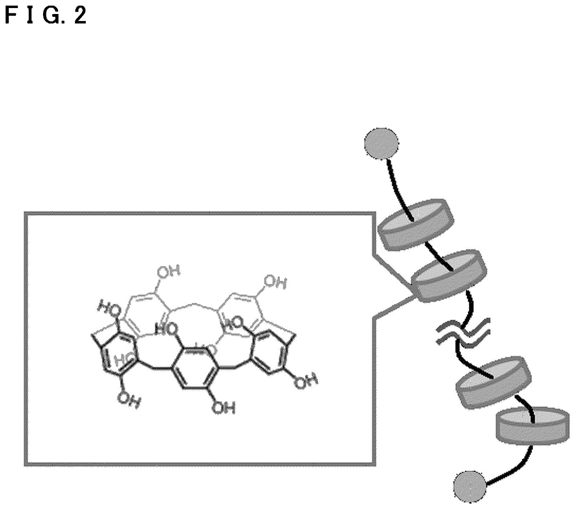

As shown in FIG. 1(4), a solution of 0.016 g (0.11 mmol) of adamantanamine, 0.048 g (0.11 mmol) of a BOP reagent, and 0.019 mL (0.12 mmol) of diisopropylethylamine in 10 mL of dimethylformamide (dry DMF) was sufficiently cooled with ice, and 150 mg of Pseudo P5AOH-PEG was added to the solution, followed by stirring at 4°C all day and night. The resultant solution was concentrated under reduced pressure with a rotary evaporator, and excess water was added to the concentrate, followed by stirring. The resultant precipitate was subjected to filtration, and acetone was added to the resultant residue, followed by ultrasonic washing, removal of the supernatant, and vacuum drying, to thereby produce 104 mg of a polyrotaxane of Example containing adamantane groups disposed at both ends of PEG (hereinafter abbreviated as "P5AOH-PEG"). FIG. 2 shows a schematic view of the P5AOH-PEG (also the structural formula of P5AOH).

### [Comparative Example]

A polyrotaxane of Comparative Example was produced from the PEG-COOH prepared in (1) above and commercially available α-cyclodextrin (abbreviated as "CD") by the method described below.

### (i) Synthesis of Polyrotaxane of Comparative Example

According to the method described in the literature (Macromolecules, 2005, 38, 7524-7527), 3.0 g (8.6 × 10⁻⁵ mol) of PEG-COOH and 12 g (1.2 × 10⁻² mol) of α-cyclodextrin were dissolved in 100 mL of water, and the solution was allowed to stand still in a refrigerator all night. The resultant paste-like mixture was freeze-dried, and the dried solid was dissolved in 100 mL of DMF together with 0.16 g (1.1 × 10⁻³ mol) of adamantanamine, 0.48 g (1.1 × 10⁻³ mol) of a BOP reagent, and 0.19 mL (1.2 × 10⁻³ mol) of ethyldiisopropylamine, followed by reaction at 4°C all day and night. The resultant mixture was subjected to centrifugation twice with a mixed solvent of DMF/MeOH (1 : 1) and twice with MeOH. The recovered precipitate was washed with 80 mL of DMSO, and 800 mL of H₂O was added to the resultant precipitate, followed by centrifugation. The resultant solid was freeze-dried to thereby produce 9.55 g to 10.3 g of a polyrotaxane of Comparative Example (hereinafter abbreviated as "CD polyrotaxane"). FIG. 3 shows a schematic view of the CD polyrotaxane (also the structural formula of CD).

### [Measurement]

The following measurements were performed on the polyrotaxanes of Example and Comparative Example.

### (A) NMR Measurement and GPC Measurement (Identification of Molecular Structure)

The polyrotaxane of Example produced in (4) above was dissolved in Acetone-d6, and the solution was subjected to ¹H-NMR measurement at 25°C with a nuclear magnetic resonance (NMR) apparatus (JEOL JNM-ECS400 and JNM-ECZ500R spectrometers), to thereby determine the presence of a peak attributed to pillararene and a peak attributed to PEG. In addition, the sample was dissolved in DMF, and the molecular weight was measured with a gel permeation chromatography (GPC) apparatus (high-performance GPC available from TOSOH), to thereby determine the production of a target structure (polyrotaxane) in which P5AOH encloses PEG.

The molecular structure of the CD polyrotaxane of Comparative Example produced in (i) above was identified by ¹H-NMR measurement.

### (B) TG-DTA Measurement (Determination of Improved Heat Resistance of Polyrotaxane of Example)

TG-DTA measurement was performed on the P5AOH-PEG of Example produced in (4) above and the CD polyrotaxane of Comparative Example produced in (i) above. For reference, TG-DTA measurement was also performed on PEG 20000 used in (1) above, P5AOH intermediately produced in (2) above, and Pseudo P5AOH-PEG intermediately produced in (3) above.

Specifically, measurement was performed with a thermogravimetric-differential thermal analysis (TG-DTA) simultaneous measuring apparatus (STA 7200, available from Hitachi High-Tech Corporation) using a platinum sample pan in a stream of N₂ gas (10 mL/minute) at a thermal decomposition temperature increase rate of 100 to 300°C • • • 1°C/minute, 300 to 900°C • • • 10°C/minute. The weight before heating was taken as baseline (100%), and the temperature at which a decrease in weight was 50% with respect to the weight before heating was defined as the thermal decomposition temperature (50% decrease in weight). The results are shown in Table 2.

Furthermore, the P5AOH-PEG of Example and the CD polyrotaxane of Comparative Example were subjected to TG-DTA measurement in a stream of dry air (10 mL/minute) under the aforementioned conditions. The weight before heating was taken as baseline (100%), and the temperature at which a decrease in weight was 5% with respect to the weight before heating was defined as the temperature at 5% decrease in weight. Similarly, the temperature at which a decrease in weight was 10% with respect to the weight before heating was defined as the temperature at 10% decrease in weight. The results are shown in Table 2.

**[Table 2]**

| TG-DTA | | Reference | | | Example | Comparative Example |
|---|---|---|---|---|---|---|
| | | PEG (20000) | P5AOH | Pseudo P5AOH-PEG | P5AOH-PEG | CD-PEG |
| During heating in a stream of N₂ gas | Thermal Decomposition Temperature [°C] (50% decrease in weight) | 395.5 | 447.1 | 467.7 | 634.6 | 434.1 |
| During heating in a stream of dry air | Temperature [°C] at 5% decrease in weight | - | - | - | 246.8 | 197.7 |
| | Temperature [°C] at 10% decrease in weight | - | - | - | 262.5 | 235.8 |

The P5AOH-PEG of Example produced in (4) above can be formed into a crosslinked polyrotaxane wherein cyclic molecules of a plurality of adjacent P5AOH-PEGs are crosslinked together with a crosslinking agent.

The crosslinked polyrotaxane can be used alone as an elastomer of high heat resistance, or can be mixed with an additional elastomer and used as an elastomer of high heat resistance.

Alternatively, the P5AOH-PEG of Example can be mixed with an additional elastomer, and cyclic molecules of the P5AOH-PEG can be directly crosslinked to a functional group of the additional elastomer, or can be crosslinked to the functional group with a crosslinking agent. The resultant crosslinked product can be used as an elastomer of high heat resistance.

Such an elastomer of high heat resistance can be attached to electrodes (e.g., stretchable electrode layers are attached to both surfaces of a film-like elastomer), and the resultant product can be used as a polymer actuator or polymer sensor of high heat resistance.

The present invention is not limited to the aforementioned examples, and may be appropriately modified and embodied without departing from the spirit of the invention.

## Claims

1. A polyrotaxane comprising a linear molecule, a cyclic molecule enclosing the linear molecule such that the cyclic molecule is skewered with the linear molecule, and blocking groups disposed at both ends of the linear molecule, **characterized in that**:
the cyclic molecule contains an aromatic ring having, on a side chain, a phenolic hydroxyl group.

2. A method for producing a polyrotaxane, the method comprising:
a step of dissolving, in a methanol-containing solvent, a linear molecule and a cyclic molecule containing an aromatic ring having, on a side chain, a phenolic hydroxyl group, to yield a pseudo polyrotaxane in which the cyclic molecule encloses the linear molecule such that the cyclic molecule is skewered with the linear molecule; and
a step of dissolving the pseudo polyrotaxane and a blocking group material in a solvent, to dispose blocking groups at both ends of the linear molecule.

3. The method for producing a polyrotaxane according to claim 2, wherein the methanol-containing solvent is a mixed solvent of methanol and water.

4. A crosslinked polyrotaxane produced through crosslinking between cyclic molecules of a plurality of the polyrotaxanes according to claim 1.

5. An elastomer comprising the crosslinked polyrotaxane according to claim 4.
